# EUROPEAN PATENT SPECIFICATION

(11) **EP 0 583 806 B1**
(45) Date of publication and mention of the grant of the patent: **15.10.1997**
(21) Application number: 93200326.2
(22) Date of filing: 05.02.1993
(51) Int. Cl.: C07D 417/04, C07D 417/14, A01N 43/78

(54) **Acrylic derivatives of 2-thiazolypyrroles having a fungicidal activity**
2-Thiazolylpyrroleakrylate mit fungizider Wirkung
2-thiazolylpyrrole acrylates ayant une activité fongicide

(30) Priority: 06.02.1992 IT MI920227
(43) Date of publication of application: 23.02.1994
(73) Proprietor: MINISTERO DELL' UNIVERSITA' E DELLA RICERCA SCIENTIFICA E TECNOLOGICA, 00196 Roma (IT)
(72) Inventor: Camaggi, Giovanni, I-28100 Novara (IT); Filippini, Lucio, I-20097 San Donato Milanese (Milan) (IT); Gusmeroli, Marilena, I-20052 Monza (Milan) (IT); Meazza, Giovanni, I-21047 Saronno (Varese) (IT); Riva, Raul, I-28100 Novara (IT); Zanardi, Giampaolo, I-27029 Vigevano (Pavia) (IT); Garavaglia, Carlo, 20012 Cuggiono (Milano) (IT); Mirenna, Luigi, 20139 Milano (IT)
(74) Representative: Fusina, Gerolamo

(56) References cited:
- EP-A- 0 273 572
- EP-A- 0 402 246

## Description

The present invention relates to compounds based on acrylic derivatives of 2-thiazol-2-ylpyrroles.

More specifically the present invention relates to compounds based on acrylic derivatives of 2-thiazol-2-ylpyrroles having a high antifungal activity, a procedure for their preparation and their use in the agricultural field as fungicides.

The present invention consequently relates to compounds based on acrylic derivatives of 2-thiazol-2-ylpyrroles having the general formula (I): 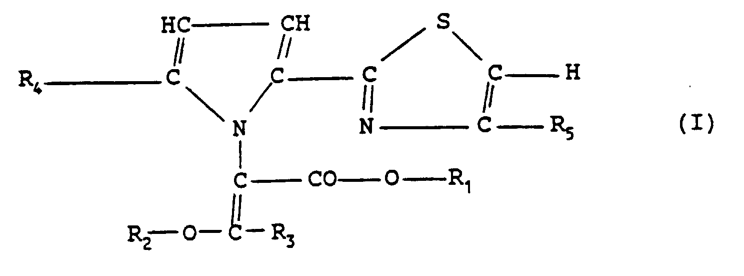 wherein:
- R₁, R₂ and R₄, the same or different, represent a hydrogen atom, or a C₁-C₆ alkyl or haloalkyl group, either linear or branched;
- R₃ represents a hydrogen atom or, jointly with R₁, a methylene;
- R₅ represents a hydrogen atom, a C₁-C₆ alkyl group, linear or branched, a phenyl group, a C₁-C₄ alkoxyl group, a phenoxyl group, a C₄-C₆ heterocycloxylic group, optionally substituted with halogens, such as chlorine, bromine, iodine, or with C₁-C₄ alkyl radicals.

The structure of general formula (I), when R₃ represents hydrogen, may have at least one E/Z isomerism.

The products having general formula (I) are antifungal for agricultural purposes.

Examples of R₁,R₂ and R₄ radicals are: methyl, ethyl, trifluoromethyl, 1,1,2,2-tetrafluoroethyl, etc. Examples of R₅ radicals are: methyl, isopropyl, ter-butyl, trifluoromethyl, perfluorobutyl, methoxyl, ethoxyl, trifluoromethoxyl, 1,1,2,2-tetrafluoroethoxyl, phenyl, 4-chlorophenyl, 2,4-dichlorophenyl, 3,4-dichlorophenyl, 2,4,6-trichlorophenyl, 3-trifluoromethylphenyl, 4-trifluoromethylphenyl, 4-fluorophenyl, 2,4-dichlorophenyl, 4-chlorophenoxyl, 2,4-dichlorophenoXyl, 2,4,6-trichlorophenoxyl, 3,5-dichloropyridoxy-2-yl, 5-trifluoromethylpyridoxy-2-yl, etc.

Compounds having general formula (I) not illustrated in the examples, but equally interesting for their fungicidal activity are: (Z)-3-methoxy-2[(4-(2,4-dichlorophenyl)thiazol-2-yl)pyrrol-1-yl] methyl acrylate, (Z)-3 -methoxy-2[(4-(isopropyl)thiazol-2-yl)pyrrol-l-yl]methyl acrylate, (Z)-3-methoxy-2[(4-(trifluoromethyl)thiazol-2-yl)pyrrol-l-yl]methyl acrylate, (Z)-3-methoxy-2[(4-(1,1,2,2-tetrafluoro ethoxy)thiazol-2-yl)pyrrol-1-yl]methyl acrylate, (Z)-3-methoxy-2[(4-(2,4-difluorophenyl)thiazol-2-yl)pyrrol-1-yl]methyl acrylate, (Z)-3-methoxy-2[(4-(3-trifluoromethylphenyl)thiazol-2-yl)pyrrol-1-yl]methyl acrylate, (Z)-3-methoxy-2[(4-(4-trifluoromethylphenyl thiazol-2-yl)pyrrol-1-yl]methyl acrylate, (Z)-3-methoxy-2[(4-(4-chlorophenoxy)thiazol-2-yl)pyrrol-1-yl]methyl acrylate, (Z)-3-methoxy-2[(4-(3,5-dichloropyridoxy-2-yl)thiazol-2-yl)pyrrol-l-yl]methyl acrylate; or 4-methoxy-3-[(4-(2,4-dichlorophenyl)thiazol-2-yl-)-pyrrol-1-yl]-(5H)-furan-2-one, 4-methoxy-3-[(4-(n-butyl)thiazol-2-yl-)-pyrrol-1-yl]-(5H)-furan-2-one, 4-methoxy-3-[(4-(trifluoromethyl)thiazol-2-yl-)-pyrrol-l-yl]-(5H)-furan-2-one, 4-methoxy-3-[(4-(trifluoromethoxy)thiazol-2-yl-)-pyrrol-1-yl]-(5H)-furan-2-one, 4-methoxy-3-[(4-(trifluoromethylphenyl)thiazol-2-yl-)-pyrrol-1-yl]-(5H)-furan-2-one, 4-methoxy-3-[(4-(4-fluorophenyl)thiazol-2-yl-)-pyrrol-1-yl]-(5H)-furan-2-one, 4-methoxy-3-[(4-(2,4-dichlorophenoxy)thiazol-2-yl-)-pyrrol-1-yl]-(5H)-furan-2-one,4-methoxy-3-[(4-(5-trifluoromethylpyridoxy-2-yl)thiazol-2-yl-)-pyrrol-1-yl]-(5H)-furan-2-one, etc.

When R₃ represents a hydrogen atom the compounds of the present invention can be obtained by means of a procedure which includes reacting a thiazole derivative having general formula (II): 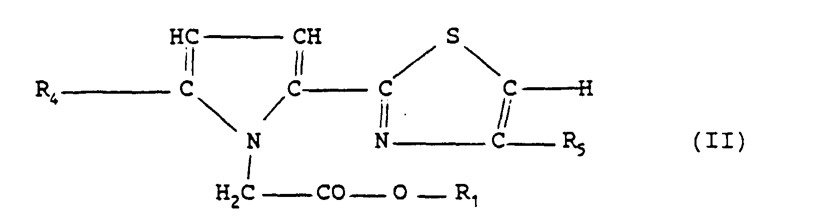 wherein R₁, R₄ and R₅ have the meaning described above, with a formiate having general formula (III): 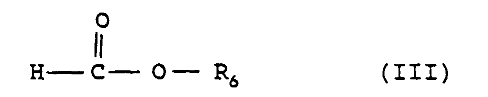 wherein R₆ represents a C₁-C₄ alkyl group, in an ether solvent, such as ethyl ether, and in the presence of a base, such as sodium hydride or potassium ter-butylate, at a temperature ranging from -10°C to 30°C, to obtain the salt having general formula (IV): 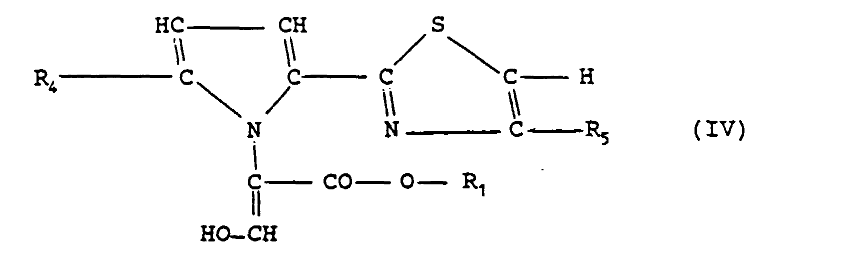 which is subsequently reacted with an alkylating agent R₂-Y, wherein R₂ has the meaning described above and Y represents a halogen atom, such as chlorine, bromine, iodine, or an activated ester, such as paratoluensulphonate, at a temperature ranging from -10°C to 30°C thus obtaining the compound having general formula (I).

When R₃ jointly with R₁ represents a methylene, the compounds of the present invention can be obtained by means of a procedure which includes reacting a thiazole derivative having formula (V): 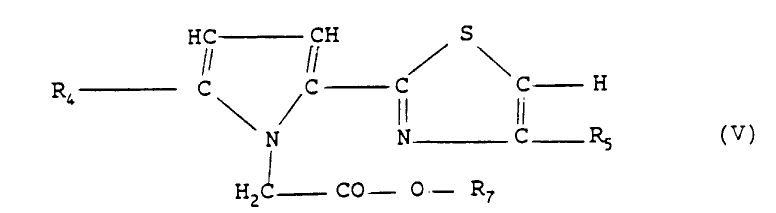 wherein R₄ and R₅ have the meaning described above and R₇ represents a -CH₂-CO-O-tC₄H₉ group, in an ether solvent, such as tetrahydrofuran, and in the presence of a base, such as sodium hydride or potassium terbutylate, at a temperature ranging from -10°C to 30°C, to obtain the salt having general formula (VI): 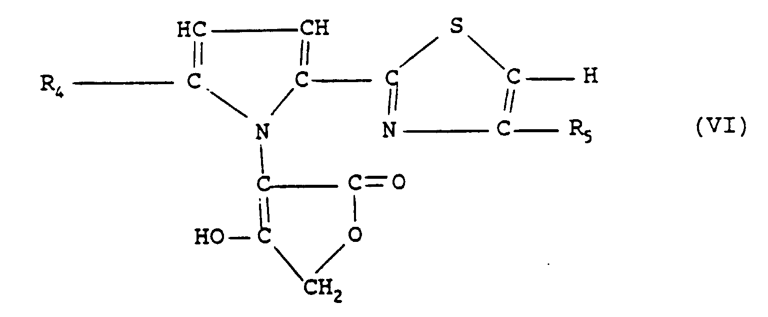 wherefrom the compound having general formula (I) is obtained, using the same procedure as described above for the transformation of the salt having general formula (IV).

The compounds having general formula (II) and (V) can be prepared with the methods described in literature such as for example in: "The chemistry of heterocyclic compounds: thiazole and its derivatives", vol. 34, V. Metzger (Ed). Wiley, (1979).

The compounds corresponding to general formula (I) have a particularly high fungicidal activity against phytopathogenic fungi which attack cultivations of vines, cereals, Cocurbitacee and fruit-trees.

They have both a preventive and curative activity when applied to useful plants or their parts, such as leaves, and are particularly effective in preventing diseases caused by obligate pathogenic fungi, such as, for example, those belonging to the species Erysiphe and Helminthosporium.

Plant diseases which can be fought with the compounds of the present invention are, for example, the following:
- Helminthosporium of cereals;
- Plasmopara viticola of vines;
- Phytium of horticultural products;
- Sphaerotheca fuliginea of cocurbitacee (e.g. cucumbers);
- Septoria of cereals;
- Erysiphe graminis of cereals;
- Rhynchosporium of cereals;
- Podosphaera leucotricha of apple-trees;
- Uncinula necator of vines;
- Venturia inequalis of apple-trees;
- Piricularia oryzae of rice;
- Botrytis cinerea;
- Fusarium of cereals; etc.

The compounds corresponding to general formula (I), as well as having both a curative and preventive fungicidal action as described above, also have a limited or non-existent phytotoxicity.

For practical uses in agriculture it is often useful to have fungicidal compositions containing one or more of the compounds of general formula (I), also possibly in an isomeric form, as active substance.

These compositions can be applied on any part of the plant, for example, leaves, stems, branches and roots, or on the seeds, before sowing, or even on the soil where the plant grows.

Compositions can be used in the form of dry powders, wettable powders, emulsionable concentrates, micro-emulsions, pastes, granules, solutions, suspensions, etc.: the choice of the type of composition depends on the specific use.

The compositions are prepared according to the known methods, for example by diluting or dissolving the active substance with a solvent medium and/or solid diluent, possibly in the presence of surface-active agents.

Solid diluents, or supports, which can be used are: silica, kaolin, bentonite, talc, infusorial earth, dolomite, calcium carbonate, magnesia, chalk, clays, synthetic silicates, attapulgite, sepiolite.

Liquid diluents, apart from water naturally, which can be used are various types of solvents, such as aromatics (xylols or mixtures of alkylbenzols), chloro aromatics (chlorobenzol), paraffins (fractions of petroleum), alcohols (methanol, propanol, butanol, octanol), amines, amides (N,N'-dimethylformamide, N-methylpyrrolidone), ketones (acetone, cyclohexanone, acetophenone,isophorone, ethylamylketone), esters (isobutyl acetate).

Surface-active agents which can be used are salts of sodium, calcium or of triethanolamine of alkylsulphates, alkylsulphonates, alkylarylsulphonates, polyethoxylated alkylphenols, fatty alcohols condensed with ethylene oxide, polyoxyethylated fatty acids, polyoxyethylated esters of sorbitol, ligninsulphonates.

The compositions may also contain special additives for particular purposes, for example adhesion agents such as arabic rubber, polyvinyl alcohol, polyvinylpyrrolidone.

If desired other compatible active substances may also be added to the compositions of the present invention, such as fungicides, phytoregulators, antibiotics, weed-killers, insecticides, fertilizers.

The concentration of active substance in the above compositions may vary within a wide range, depending on the active compound, cultivation, pathogen, environmental conditions and type of formulation used.

In general the concentration of active substance varies from 0.1 to 95%, preferably from 0.5 to 90%.

The following examples provide an illustration of the present invention but do not limit it in any way.

### EXAMPLE 1

### Preparation of (Z)-3-methoxy-2-[(4-phenylthiazol-2-yl-) pyrrol-1-yl]methyl acrylate (Compound No.1).

0.6 g of sodium hydride are dispersed in 10 cm³ of anhydrous DMF, in a nitrogen atmosphere.

3.0 g of 2-[(4-phenylthiazol-2-yl-)pyrrol-1-yl] methyl acetate in 10 cm³ of methyl formiate are then added dropwise, in 30 minutes.

The mixture is kept for four hours at room temperature.

It is cooled to 5°C and 6.2 cm³ of CH₃I are added.

The solution thus obtained is concentrated at reduced pressure and the crude product obtained is purified on silica gel, using hexane:ethyl acetate in a ratio of 9:1, as eluant.

1.6 g of compound No.1 are obtained with a yield of 47%, the structure of which is shown in table 1.

Table 2 shows the NMR spectroscopic data.

### EXAMPLE 2

### Preparation of 4-methoxy-3-[(4-(4-clorophenyl)thiazol-2-yl)-pyrrol-1-yl]-(5H)-furan-2-one.

0.48 g of sodium hydride are dispersed in 10 cm³ of anhydrous DMF, in a nitrogen atmosphere.

6.75 g of [(4-(4-chlorophenyl)thiazol-2-yl-) pyrrol-1-yl]-terbutyl acetoxyacetate in 10 cm³ of anhydrous DMF are then added dropwise, in 30 minutes.

The mixture is kept for three hours at room temperature and then poured into acid water.

At this point the crude product is extracted with ethyl acetate previously anhydrified on sodium sulphate and concentrated under vacuum.

10 cm³ of a mixture of ethanol:water in a ratio 4:1 and 1.40 g of sodium bicarbonate are added to the crude product thus obtained.

When there is no more effervescence, 1.52 cm³ of methyl sulphate are added and the mixture is heated to reflux temperature for four hours.

20 cm³ of water are then added and the mixture is extracted with methylene chloride.

The solution thus obtained is concentrated at reduced pressure and the crude product is purified on silica gel, using hexane:ethyl acetate in a ratio of 7:3 as eluant.

0.90 g of compound No.6 are obtained with a yield of 15.5%, the structure of which is shown in table 1.

Table 2 shows the NMR spectroscopic data.

### EXAMPLES 3-9

Using the same procedure as for examples 1 and 2, compounds No. 2-5 and 7-9 were prepared, the structures of which are shown in table 1.

The respective spectroscopic data are shown in Table 2.

### EXAMPLE 10

### Determination of the preventive fungicidal activity against Helminthosporium teres.

Leaves of barley cultivar Arna, grown in vases in a conditioned environment, are sprayed on both sides of the leaves with compounds No. 1-9 in a 20% by volume hydroacetonic solution of acetone (the concentration of the fungicide is 200 ppm).

After remaining two days in a conditioned environment at 20°C and 70% relative humidity, the plants were sprayed on both sides of the leaves with an aqueous suspension of conidia of Helminthosporium teres (250000 conidia per cm³).

After remaining 24 hours in a humidity-saturated environment, at 21°C, the plants were kept in a conditioned environment to allow for the incubation of the fungus.

At the end of this period (12 days), complete control of the disease was obtained.

**TABLE 1**

| Compound | (1) | R¹ | R² | R³ | R⁴ | R⁵ |
|---|---|---|---|---|---|---|
| 1 | E | CH₃ | CH₃ | H | H | phenyl |
| 2 | E | CH₃ | CH₃ | H | H | 4-chlorophenyl |
| 3 | E | CH₃ | CH₃ | H | H | t-butyl |
| 4 | E | CH₃ | CH₃ | H | H | methyl |
| 5 | E | CH₃ | CH₃ | (2) | H | phenyl |
| 6 | E | CH₃ | CH₃ | (2) | H | 4-chlorophenyl |
| 7 | E | CH₃ | CH₃ | (2) | H | t-butyl |
| 8 | E | CH₃ | CH₃ | H | H | cyclopropyl |
| 9 | E | CH₃ | CH₃ | H | CH₃ | t-butyl |
| (1) = isomerism of the 3-alcoxyacrylic group (2) = R³ in conjunction with R¹ is equal to CH₂ | | | | | | |

**TABLE 2**

| Compound | NMR 60 MHz spectroscopy data (DMSO D⁶) |
|---|---|
| 1 | 7.60(1H)s, 7.60(5H)m, 7.20(1H)s, 6.80(2H)m, 6.25(1H)m, 3.80(3H)s, 3.60(3H)s. |
| 2 | 7.80(2H)m, 7.65(1H)s, 7.30(2H)m, 7.25(1H)s, 6.90(2H)m, 6.35(1H)m, 3.90(3H)s, 3.70(3H)s. |
| 3 | 7.53(1H)s, 6.72(2H)m, 6.64(1H)s, 6.28(1H)m, 3.82(3H)s, 3.66(3H)s, 1.29(9H)s. |
| 4 | 7.55(1H)s, 6.75(2H)m, 6.64(1H)s, 6.29(1H)m, 3.84(3H)s, 3.65(3H)s, 2.38(3H)d. |
| 5 | 7.65(5H)m, 7.25(1H)s, 6.80(2H)m, 6.25(1H)m, 4.55(2H)s, 3.50(3H)s. |
| 6 | 7.75(2H)m, 7.25(2H)m, 7.10(1H)s, 6.6(2H)m, 6.10(1H)m, 4.50(2H)s, 3.50(3H)s. |
| 7 | 6.75(2H)m, 6.64(1H)s, 6.28(1H)m, 4.50(2H)s, 3.50(1H)s, 1.29(9H)s. |
| 8 | 7.52(1H)s, 6.76(1H)m, 6.66(1H)m, 6.64(1H)s, 6.37(1H)m, 3.82(3H)s, 3.68(3H)s, 1.95(1H)m 0.90(4H)m. |
| 9 | 7.52(1H)s, 6.61(2H)m, 6.24(1H)s, 3.81(3H)s, 3.64(3H)s, 2.44(3H)s, 1.33(9H)s. |

## Claims

1. Compounds based on acrylic derivatives of 2-thiazol-2-yl pyrroles having the general formula (I): 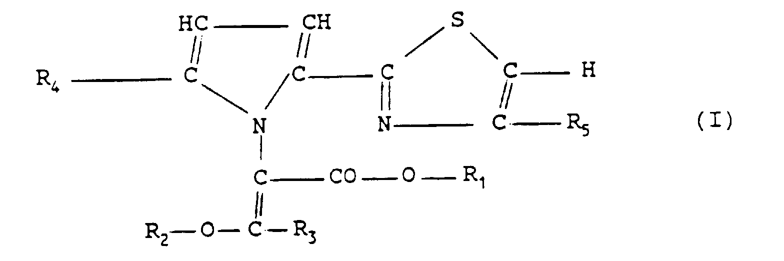 wherein:
- R₁, R₂ and R₄, the same or different, represent a hydrogen atom, or a C₁-C₆ alkyl or haloalkyl group, either linear or branched;
- R₃ represents a hydrogen atom or, jointly with R₁, a methylene;
- R₅ represents a hydrogen atom, a C₁-C₆ alkyl group, linear or branched, a phenyl group, a C₁-C₄ alkoxyl group, a phenoxyl group, a C₄-C₆ heterocycloxylic group, optionally substituted with halogens, such as chlorine, bromine, iodine, or with C₁-C₄ alkyl radicals.

2. Antifungal agents for agricultural purposes composed of compounds based on acrylic derivatives of 2-thiazol-2-ylpyrroles having general formula (I): 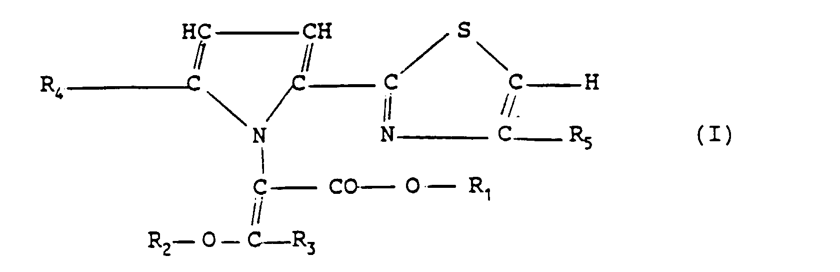 wherein:
- R₁, R₂ and R₄, the same or different, represent a hydrogen atom, or a C₁-C₆ alkyl or haloalkyl group, either linear or branched;
- R₃ represents a hydrogen atom or, jointly with R₁, a methylene;
- R₅ represents a hydrogen atom, a C₁-C₆ alkyl group, linear or branched, a phenyl group, a C₁-C₄ alkoxyl group, a phenoxyl group, a C₄-C₆ heterocycloxylic group, optionally substituted with halogens, such as chlorine, bromine, iodine, or with C₁-C₄ alkyl radicals.

3. Antifungal agents for agricultural purposes according to claim 2, wherein R₁, R₂ and R₄ are: methyl, ethyl, trifluoromethyl, 1,1,2,2-tetrafluoroethyl.

4. Antifungal agents for agricultural purposes according to claim 2, wherein R₅ is: methyl, isopropyl, ter-butyl, trifluoromethyl, perfluorobutyl, methoxyl, ethoxyl, trifluoromethoxyl, 1,1,2,2-tetrafluoroethoxyl, phenyl, 4-chlorophenyl, 2,4-dichlorophenyl, 3,4-dichlorophenyl, 2,4,6-trichlorophenyl, 3-trifluoromethylphenyl, 4-trifluoromethylphenyl, 4-fluorophenyl, 2,4-dichlorophenyl, 4-chlorophenoxyl, 2,4-dichlorophenoxyl, 2,4,6-trichlorophenoxyl, 3,5-dichloropyridoxy-2-yl, 5-trifluoromethylpyridoxy-2-yl.

5. Antifungal agent for agricultural purposes according to claim 2, composed of (Z)-3-methoxy-2-[(4-phenylthiazol-2-yl-)pyrrol-1-yl]methyl acrylate.

6. Antifungal agent for agricultural purposes according to claim 2, composed of (Z)-3-methoxy-2-[(4-(4-chlorophenyl)thiazol-2 -yl-)pyrrol-1-yl]methyl acrylate.

7. Antifungal agent for agricultural purposes according to claim 2, composed of (Z)-3-methoxy-2-[(4-terbutylthiazol-2-yl-)pyrrol-1-yl]methyl acrylate.

8. Antifungal agent for agricultural purposes according to claim 2, composed of 4-methoxy-3-[(4-phenylthiazol-2-il)-pyrrol-1-yl]-(5H)-furan-2-one.

9. Antifungal agent for agricultural purposes according to claim 2, composed of 4-methoxy-3-[(4-(4-chlorophenyl)thiazol-2-yl-)pyrrol-1-yl]-(5H)-furan-2-one.

10. Antifungal agent for agricultural purposes according to claim 2, composed of 4-methoxy-3-[(4-terbutylphenyl)thiazol-2-yl-)pyrrol-1-yl]-(5H)-furan-2-one.

11. Antifungal agent for agricultural purposes according to claim 2, composed of (Z)-3-methoxy-2-[(4-cyclopropylthiazol-2-yl-)pyrrol-1-yl]methyl acrylate.

12. Antifungal agent for agricultural purposes according to claim 2, composed of (Z)-3-methoxy-2-[(4-terbutyl-5-methylthiazol-2-yl-)pyrrol-1-yl] methyl acrylate.

13. Procedure for the preparation of the compounds according to any of the previous claims, which includes reacting a thiazole derivative having general formula (II): 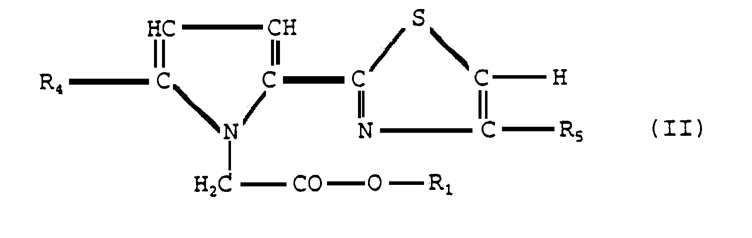 wherein R₁, R₄ and R₅ have the meaning described above, with a formiate having general formula (III): 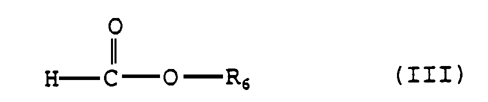 wherein R₆ represents a C₁-C₄ alkyl group, in an ether solvent and in the presence of a base, at a temperature ranging from -10°C to 30°C, to obtain the salt having general formula (IV): 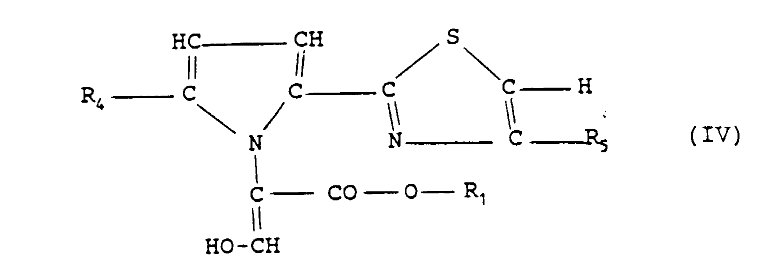 which is subsequently reacted with an alkylating agent R₂-Y, wherein R₂ has the meaning described above and Y represents a halogen atom, or an activated ester, at a temperature ranging from -10°C to 30°C thus obtaining the compound having general formula (I).

14. Procedure for the preparation of the compounds according to any of the previous claims, which includes reacting a thiazole derivative having formula (V): 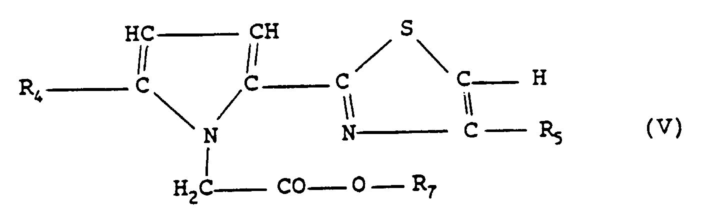 wherein R₄ and R₅ have the meaning described above and R₇ represents a -CH₂-CO-O-tC₄H₉ group, in an ether solvent and in the presence of a base, at a temperature ranging from -10°C to 30°C, to obtain the salt having general formula (VI): 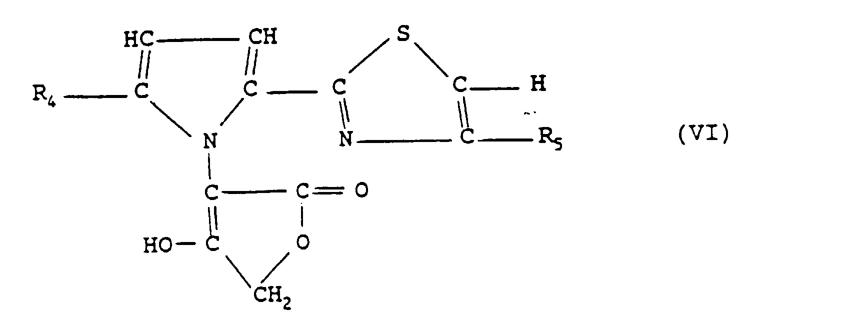 wherefrom the compound having general formula (I) is obtained, using the same procedure as described in claim 13 for the transformation of the salt having general formula (IV).

15. Fungicidal compositions containing one or more of the compounds according to any of the claims from 2 to 14, alone or in the presence of solid supports, liquid diluents, surface-active agents or other active principles.

16. Method for fighting fungal infections consisting in applying the fungicidal compositions according to claim 15 on the plants, leaves, stems, branches and roots, or on the seeds before sowing, or on the soil where the plant grows.

## Patentansprüche

1. Verbindungen auf der Grundlage von Acrylderivaten von 2-Thiazol-2-yl-pyrrolen der allgemeinen Formel (I) 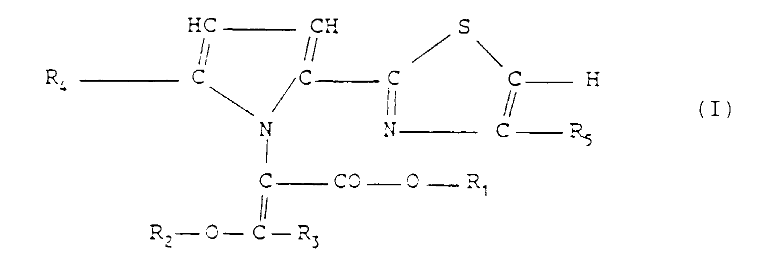 worin
- R₁, R₂ und R₄, die gleich oder unterschiedlich sein können, ein Wasserstoffatom oder eine C₁-C₆-Alkyl- oder Halogenalkylgruppe, entweder linear oder verzweigt, bedeuten;
- R₃ ein Wasserstoffatom oder zusammen mit R₁ Methylen bedeutet;
- R₅ ein Wasserstoffatom, eine lineare oder verzweigte C₁-C₆-Alkylgruppe, eine Phenylgruppe, eine C₁-C₄-Alkoxylgruppe, eine Phenoxylgruppe, eine C₄-C₆-heterocyclische Oxygruppe, gegebenenfalls substituiert mit Halogenen, wie mit Chlor, Brom, Iod oder mit C₁-C₄-Alkylgruppen, bedeutet.

2. Antifungale Mittel für landwirtschaftliche Zwecke, zusammengesetzt aus Verbindungen auf der Grundlage von Acrylderivaten von 2-Thiazol-2-yl-pyrrolen der allgemeinen Formel (I): 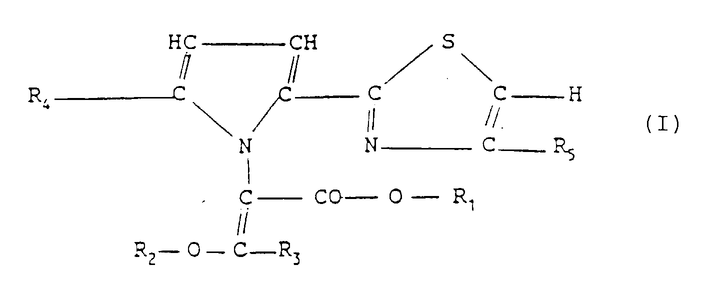 worin:
- R₁, R₂ und R₄, die gleich oder unterschiedlich sein können, ein Wasserstoffatom oder eine C₁-C₆-Alkyl- oder Halogenalkylgruppe, entweder linear oder verzweigt, bedeuten;
- R₃ ein Wasserstoffatom oder zusammen mit R₁ Methylen bedeutet;
- R₅ ein Wasserstoffatom, eine lineare oder verzweigte C₁-C₆-Alkylgruppe, eine Phenylgruppe, eine C₁-C₄-Alkoxylgruppe, eine Phenoxylgruppe, eine C₄-C₆-heterocyclische Oxygruppe, gegebenenfalls substituiert mit Halogenen, wie mit Chlor, Brom, Iod oder mit C₁-C₄-Alkylgruppen, bedeutet.

3. Antifungale Mittel für landwirtschaftliche Zwecke nach Anspruch 2, dadurch **gekennzeichnet,** daß R₁, R₂ und R₄ Methyl, Ethyl, Trifluormethyl, 1,1,2,2-Tetrafluorethyl bedeuten.

4. Antifungale Mittel für landwirtschaftliche Zwecke nach Anspruch 2, dadurch **gekennzeichnet,** daß R₅ Methyl, Isopropyl, Terbutyl, Trifluormethyl, Perfluorbutyl, Methoxyl, Ethoxyl, Trifluormethoxyl, 1,1,2,2-Tetrafluorethoxyl, Phenyl, 4-Chlorphenyl, 2,4-Dichlorphenyl, 3, 4-Dichlorphenyl, 2,4, 6-Trichlorphenyl, 3-Trifluormethylphenyl, 4-Trifluormethylphenyl, 4-Fluorphenyl, 2,4-Dichlorphenyl, 4-Chlorphenoxyl, 2,4-Dichlorphenoxyl, 2,4,6-Trichlorphenoxyl, 3,5-Dichlorpyridoxy-2-yl, 5-Trifluormethylpyridoxy-2-yl bedeutet.

5. Antifungales Mittel für landwirtschaftliche Zwecke nach Anspruch 2, dadurch **gekennzeichnet,** daß es (Z)-3-Methoxy-2-[(4-phenylthiazol-2-yl) pyrrol-1-yl]methylacrylat umfaßt.

6. Antifungales Mittel für landwirtschaftliche Zwecke nach Anspruch 2, dadurch **gekennzeichnet,** daß es (Z)-3-Methoxy-2-[(4-(4-chlorphenyl)thiazol-2-yl)pyrrol-1-yl]methylacrylat umfaßt.

7. Antifungales Mittel für landwirtschaftliche Zwecke nach Anspruch 2, dadurch **gekennzeichnet,** daß es (Z)-3-Methoxy-2-[(4-terbutylthiazol-2-yl)pyrrol-1-yl]methylacrylat umfaßt.

8. Antifungales Mittel für landwirtschaftliche Zwecke nach Anspruch 2, dadurch **gekennzeichnet,** daß es 4-Methoxy-3-[(4-phenylthiazol-2-yl)pyrrol-1-yl]-(5H)-furan-2-on umfaßt.

9. Antifungales Mittel für landwirtschaftliche Zwecke nach Anspruch 2, dadurch **gekennzeichnet,** daß es 4-Methoxy-3 -[(4-(4-chlorphenyl)thiazol-2-yl)pyrrol-l-yl]-(5H)-furan-2-on umfaßt.

10. Antifungale Mittel für landwirtschaftliche Zwecke nach Anspruch 2, dadurch **gekennzeichnet,** daß es 4-Methoxy-3-[(4-tert.-butylphenyl)thiazol-2-yl)pyrrol-1-yl]-(5H)-furan-2-on umfaßt.

11. Antifungale Mittel für landwirtschaftliche Zwecke nach Anspruch 2, dadurch **gekennzeichnet,** daß es (Z)-3-Methoxy-2-[(4-cyclopropylthiazol-2-yl)pyrrol-1-yl]methylacrylat umfaßt.

12. Antifungale Mittel für landwirtschaftliche Zwecke nach Anspruch 2, dadurch **gekennzeichnet,** daß es (Z)-3-Methoxy-2-[(4-tert.-butyl-5-methylthiazol-2-yl)pyrrol-1-yl]methylacrylat umfaßt.

13. Verfahren zur Herstellung von Verbindungen nach einem der vorhergehenden Ansprüche durch Umsetzung eines Thiazolderivats der allgemeinen Formel (II): 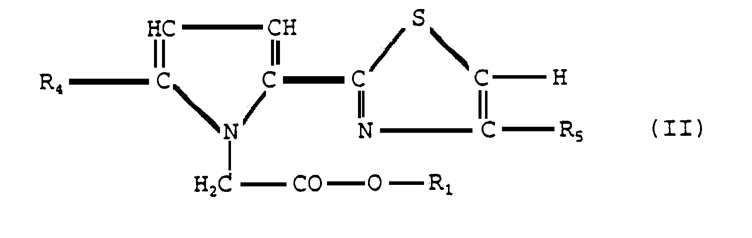 worin R₁, R₄ und R₅ die oben gegebenen Bedeutungen besitzen, mit einem Formiat der allgemeinen Formel (III): 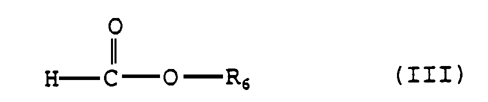 worin R₆ eine C₁-C₄-Alkylgruppe bedeutet, in einem Etherlösungsmittel und in Anwesenheit einer Base bei einer Temperatur im Bereich von -10°C bis 30°C unter Bildung eines Salzes der allgemeinen Formel (IV): 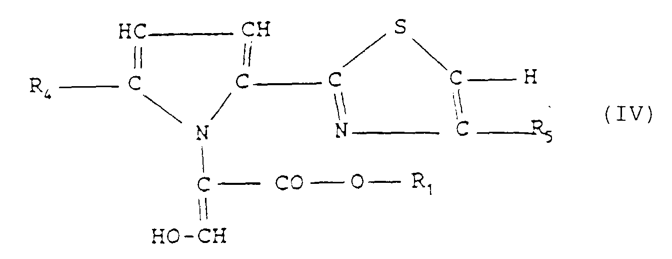 anschließende Umsetzung dieses Salzes mit einem Alkylierungsmittel R₂-Y, worin R₂ die oben gegebene Bedeutung besitzt und Y ein Halogenatom bedeutet, oder mit einem aktivierten Ester bei einer Temperatur im Bereich von -10°C bis 30°C, wobei eine Verbindung der allgemeinen Formel (I) erhalten wird.

14. Verfahren zur Herstellung von Verbindungen nach einem der vorhergehenden Ansprüche durch Umsetzung eines Thiazolderivats der Formel (V): 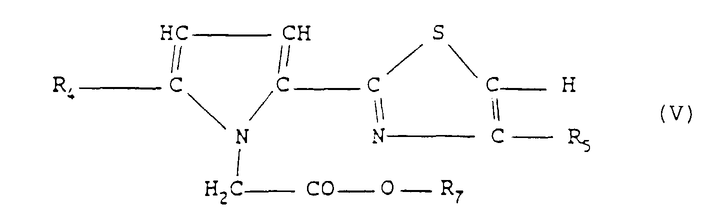 worin R₄ und R₅ die oben gegebenen Bedeutungen besitzen und R₇ eine -CH₂-CO-O-tC₄H₉-Gruppe bedeutet, in einem Etherlösungsmittel in Anwesenheit einer Base bei einer Temperatur im Bereich von -10°C bis 30°C unter Bildung eines Salzes der allgemeinen Formel (VI): 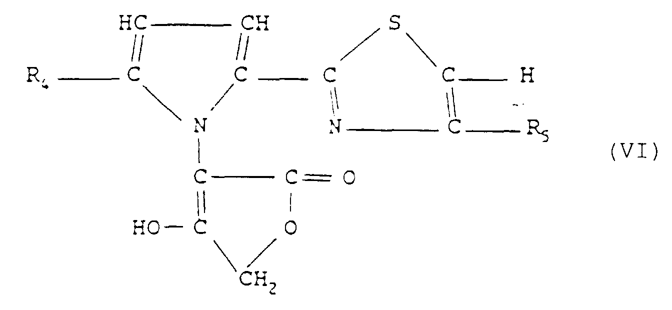 aus dem die Verbindung der allgemeinen Formel (I) unter Verwendung des gleichen Verfahrens, wie es in Anspruch 13 für die Umwandlung des Salzes der allgemeinen Formel (IV) beschrieben wurde, erhalten wird.

15. Fungizide Zusammensetzung, enthaltend eine oder mehrere der Verbindungen nach einem der Ansprüche 2 bis 14, alleine oder in Anwesenheit fester Träger, flüssiger Verdünnungsmittel, grenzflächenaktiver Mittel oder anderer aktiver Prinzipien.

16. Verfahren zur Bekämpfung von Pilzinfektionen durch Anwendung der fungiziden Zusammensetzung nach Anspruch 15 auf die Pflanzen, Blätter, Stämme, Zweige und Wurzeln oder auf die Samen vor dem Säen oder auf den Boden, wo die Pflanzen wachsen.

## Revendications

1. Composés à base de dérivés acryliques de 2-thiazol-2-yl-pyrroles de formule générale (I) : 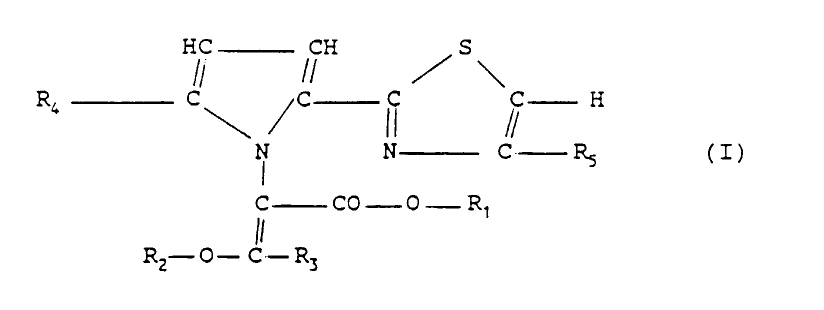 dans laquelle :
- R₁, R₂ et R₄, qui sont identiques ou différents, représentent chacun un atome d'hydrogène ou un groupe alkyle ou halogénoalkyle en C₁ à C₆ à chaîne droite ou ramifiée ;
- R₃ représente un atome d'hydrogène ou, conjointement à R₁, un groupe méthylène ;
- R₅ représente un atome d'hydrogène, un groupe alkyle en C₁ à C₆ à chaîne droite ou ramifiée, un groupe phényle, un groupe alcoxy en C₁ à C₄, un groupe phénoxy, un groupe hétérocycloxylique en C₄ à C₆, éventuellement substitué par des atomes d'halogène tels que le chlore, le brome et l'iode, ou par des radicaux alkyle en C₁ à C₄.

2. Agents antifongiques à usage agricole composés de composés à base de dérivés acryliques de 2-thiazole-2-yl-pyrroles de formule générale (I) : 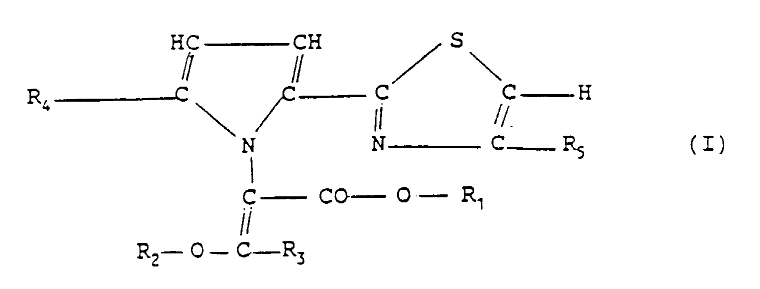 dans laquelle :
- R₁, R₂ et R₄, qui sont identiques ou différents, représentent chacun un atome d'hydrogène ou un groupe alkyle ou halogénoalkyle en C₁ à C₆ à chaîne droite ou ramifiée ;
- R₃ représente un atome d'hydrogène ou, conjointement à R₁, un groupe méthylène ;
- R₅ représente un atome d'hydrogène, un groupe alkyle en C₁ à C₆ à chaîne droite ou ramifiée, un groupe phényle, un groupe alcoxy en C₁ à C₄, un groupe phénoxy, un groupe hétérocycloxylique en C₄ à C₆, éventuellement substitué par des atomes d'halogène tels que le chlore, le brome et l'iode, ou par des radicaux alkyle en C₁ à C₄.

3. Agents antifongiques à usage agricole selon la revendication 2, dans lesquels R₁, R₂ et R₄ sont des radicaux méthyle, éthyle, trifluorométhyle ou 1,1,2,2-tétrafluoroéthyle.

4. Agents antifongiques à usage agricole selon la revendication 2, dans lesquels R₅ est un radical méthyle, isopropyle, tert-butyle, trifluorométhyle, perfluo-robutyle, méthoxy, éthoxy, trifluorométhoxy, 1,1,2,2-tétrafluoroéthoxy, phényle, 4-chlorophényle, 2,4-di-chlorophényle, 3,4-dichlorophényle, 2,4,6-trichlorophényle, 3-trifluorométhylphényle, 4-trifluorométhylphényle, 4-fluorophényle, 2,4-dichlorophényle, 4-chlorophénoxy, 2,4-dichlorophénoxy, 2,4,6-trichlorophénoxy, 3,5-dichloropyridoxy-2-yle, ou 5-trifluorométhylpyridoxy-2-yle.

5. Agent antifongique à usage agricole selon la revendication 2, composé d'acrylate de (Z)-3-méthoxy-2-[(4-phénylthiazole-2-yl)pyrrole-1-yl]méthyle.

6. Agent antifongique à usage agricole selon la revendication 2, composé d'acrylate de (Z)-3-méthoxy-2-[(4-(4-chlorophényl)thiazole-2-yl)pyrrole-1-yl]méthyle.

7. Agent antifongique à usage agricole selon la revendication 2, composé d'acrylate de (Z)-3-méthoxy-2-[(4-tert-butylthiazole-2-yl)pyrrole-1-yl]méthyle.

8. Agent antifongique à usage agricole selon la revendication 2, composé de 4-méthoxy-3-[(4-phénylthiazole-2-yl)pyrrole-1-yl]-(5H)-furanne-2-one.

9. Agent antifongique à usage agricole selon la revendication 2, composé de 4-méthoxy-3-[(4-(4-chlorophényl)thiazole-2-yl)pyrrole-1-yl]-(5H)-furanne-2-one.

10. Agent antifongique à usage agricole selon la revendication 2, composé de 4-méthoxy-3-[(4-tertbutylphényl)thiazole-2-yl)pyrrole-1-yl]-(5H)-furanne-2-one.

11. Agent antifongique à usage agricole selon la revendication 2, composé d'acrylate de (Z)-3-méthoxy-2-[(4-cyclopropylthiazole-2-yl)pyrrole-1-yl]méthyle.

12. Agent antifongique à usage agricole selon la revendication 2, composé d'acrylate de (Z)-3-méthoxy-2-[(4-tert-butyl-5-méthylthiazole-2-yl)pyrrole-1-yl]méthyle.

13. Procédé pour la préparation des composés selon l'une quelconque des revendications précédentes, qui comprend la réaction d'un dérivé thiazole de formule générale (II) : 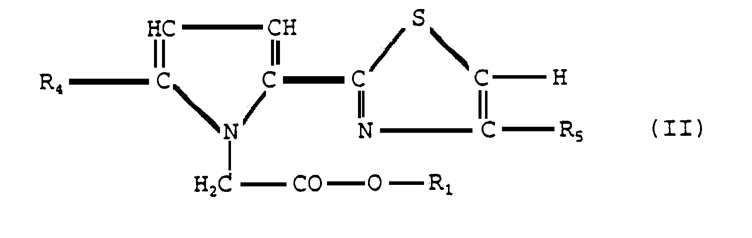 dans laquelle R₁, R₄ et R₅ ont les significations indiquées ci-dessus,
avec un formiate de formule générale (III) : 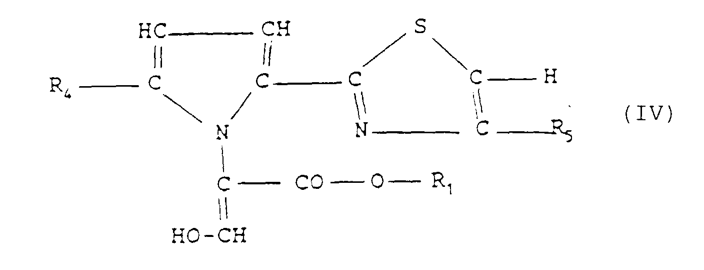 dans laquelle R₆ représente un groupe alkyle en C₁ à C₄, dans un solvant de type éther et en présence d'une base, à une température comprise entre -10°C et 30°C, pour obtenir le sel de formule générale (IV) : 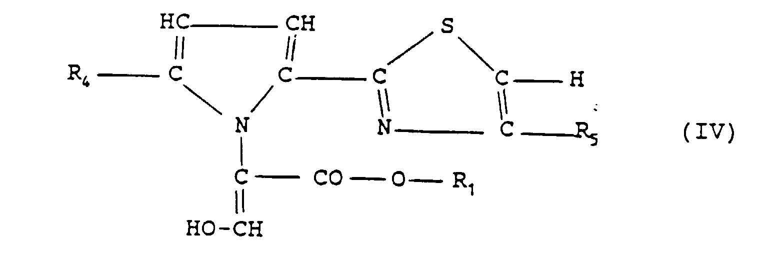 qui est ensuite mis à réagir avec un agent d'alkylation R₂-Y où R₂ a la signification indiquée ci-dessus et Y représente un atome d'halogène, ou un ester activé, à une température comprise entre -10°C et 30°C, pour obtenir le composé de formule générale (I).

14. Procédé pour la préparation des composés selon l'une quelconque des revendications précédentes, qui comprend la réaction d'un dérivé thiazole de formule (V) : 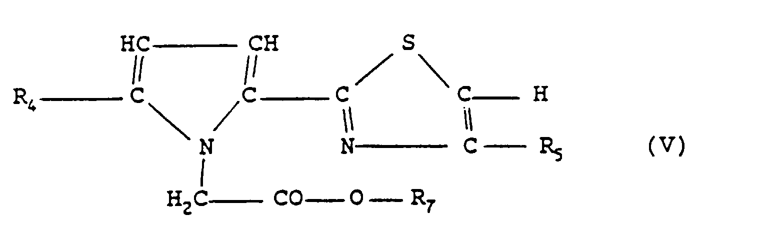 dans laquelle R₄ et R₅ ont les significations indiquées ci-dessus et R₇ représente un groupe -CH₂-CO-O-tC₄H₉, dans un solvant de type éther et en présence d'une base, à une température comprise entre -10°C et 30°C, pour obtenir le sel de formule générale (VI) : 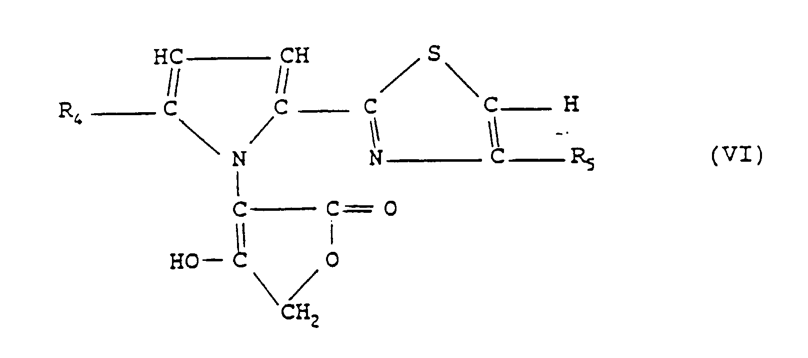 à partir duquel on obtient le composé de formule générale (I) en utilisant le même procédé que celui décrit dans la revendication 13 à propos de la transformation du sel de formule générale (IV).

15. Compositions fongicides contenant un ou plusieurs des composés selon l'une quelconque des revendications 2 à 14, seuls ou en présence de supports solides, diluants liquides, agents tensioactifs ou autres principes actifs.

16. Procédé pour combattre des infections fongiques, qui consiste à appliquer les compositions fongicides selon la revendication 15 sur des plants, feuilles, tiges, branches et racines, ou sur les graines avant semis, ou sur le sol sur lequel poussent les plantes.
